(19) Europäisches Patentamt
European Patent Office
Office européen des brevets

(11) **EP 3 844 135 B1**

(12) **EUROPEAN PATENT SPECIFICATION**

(45) Date of publication and mention
of the grant of the patent:
**24.08.2022 Bulletin 2022/34**

(21) Application number: **19783650.5**

(22) Date of filing: **29.08.2019**

(51) International Patent Classification (IPC):
***C07C 31/04*** (2006.01)     ***C01B 3/00*** (2006.01)
***C01B 3/12*** (2006.01)     ***C07C 29/151*** (2006.01)

(52) Cooperative Patent Classification (CPC):
(C-Sets available)
**C01B 3/16; C07C 29/1518;** C01B 2203/0244;
C01B 2203/0255; C01B 2203/0261;
C01B 2203/0283; C01B 2203/0405;
C01B 2203/043; C01B 2203/061; C01B 2203/1076

(Cont.)

(86) International application number:
**PCT/IB2019/057295**

(87) International publication number:
**WO 2020/044286 (05.03.2020 Gazette 2020/10)**

(54) **METHOD AND SYSTEM FOR SYNTHESIZING METHANOL**

VERFAHREN UND SYSTEM ZUR SYNTHESE VON METHANOL

PROCÉDÉ ET SYSTÈME DE SYNTHÈSE DE MÉTHANOL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.08.2018 US 201862724121 P**

(43) Date of publication of application:
**07.07.2021 Bulletin 2021/27**

(73) Proprietor: **ENI S.p.A.**
**00144 Roma (IT)**

(72) Inventor: **PANT, Atul**
**Sarjapura Bengaluru 562125 (IN)**

(74) Representative: **Bottero, Carlo et al**
**Barzanò & Zanardo Milano S.p.A.**
**Via Borgonuovo, 10**
**20121 Milano (IT)**

(56) References cited:
**US-B1- 9 969 666**

**(Cont. next page)**

(52) Cooperative Patent Classification (CPC): (Cont.)

C-Sets
**C07C 29/1518, C07C 31/04**

**Description**

TECHNICAL FIELD

The invention relates to processes and systems for synthesizing methanol.

BACKGROUND

Methanol may be synthesized by the hydrogenation of carbon oxides (carbon monoxide (CO) and carbon dioxide ($CO_2$)). US9969666, for instance, discloses a method of synthesizing methanol. In current methanol plants, the syngas feed comprising hydrogen gas and the carbon oxides that is delivered to the methanol reactor has a desired stoichiometric number typically around five. The stoichiometric number or M value gives an indication of the hydrogen available for forming methanol and is defined by Equation (1) below:

$$M = (F_{H2} - F_{CO2})/(F_{CO}+F_{CO2}). \qquad (1)$$

where $F_{H2}$, $F_{CO2}$, and $F_{CO}$ are the molar flow rates of each of $H_2$, $CO_2$, and CO, respectively, in the syngas. In the description that follows, the M value and stoichiometric number may be used interchangeably. As can be seen from Equation (1) above, a higher M value may result with higher $H_2$ concentrations and/or lower $CO_2$ concentrations. Also in the description that follows, loop M value refers to the M value in the feed gas entering the methanol reactor.

The hydrogen gas reacts with CO and $CO_2$ to form methanol and water in the reactor. From reaction stoichiometry, two moles of hydrogen are required to hydrogenate CO to methanol and three moles of hydrogen gas are required to hydrogenate $CO_2$ to methanol and water. Conversion of $CO_2$ to methanol results in loss of hydrogen to water formation. The methanol synthesis reaction is represented by the reactions of Equations (2) and (3) below:

$$CO + 2H_2 \rightarrow CH_3OH \qquad (2)$$

$$CO_2 + 3H_2 \rightarrow CH_3OH + H_2O \qquad (3)$$

The formation of methanol is equilibrium limited, which limits the conversion across the reactor. As a result, the methanol reactor is operated with a recycle loop, which sends a portion of the unreacted syngas back to reactor inlet after separating methanol.

When the syngas fed to the reactor is lean in hydrogen, the relative amount of unreacted CO and $CO_2$ are much higher at the reactor outlet. This leads to an accumulation of CO and $CO_2$ in the recycle loop relative to hydrogen, which reduces the stoichiometric or M value of the feed sent to the methanol reactor. At lower stoichiometric value, the net heat released during methanol synthesis is higher for production of similar amount of methanol and can potentially damage the catalyst. Further, the potential to form higher alcohols increases at lower stoichiometric number.

Various methods have been used to increase the hydrogen concentration to increase the stoichiometric number. This typically involves purging a large fraction of unreacted gas to recover enough hydrogen to achieve a targeted stoichiometric number in the reactor feed. The larger purge gas flow rate results in loss of unreacted carbon oxides, which reduces the carbon conversion in the loop. Furthermore, separation efficiencies often limit the hydrogen recovery process.

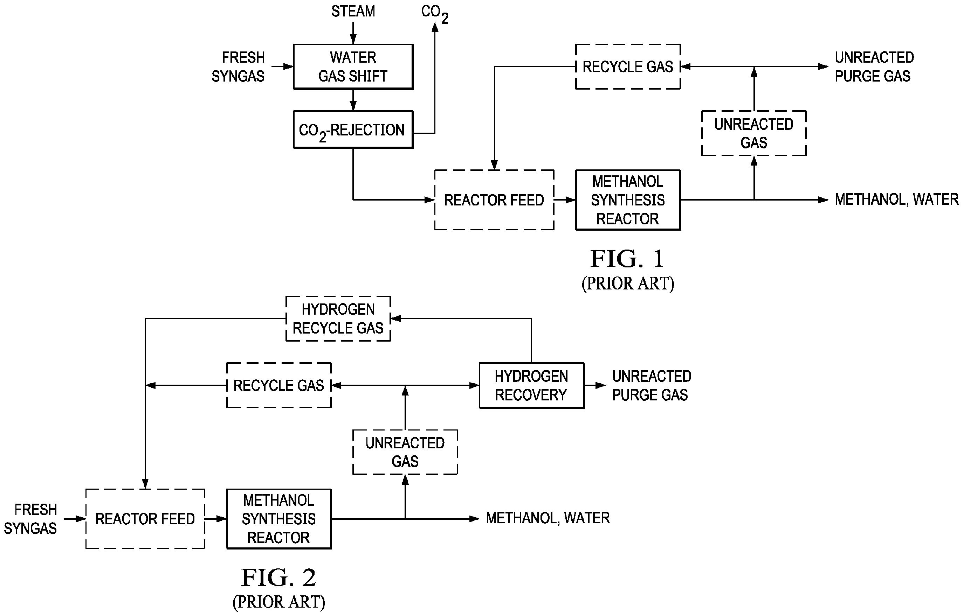

FIG. 1 shows a prior art methanol synthesis system where steam and CO in the fresh syngas are converted to hydrogen gas by a water gas shift (WGS) reaction with rejection of $CO_2$ by an appropriate separation mechanism. Such a system increases the relative concentration of hydrogen in the syngas fed to the methanol loop allowing an increase in the stoichiometric number in the feed delivered to the methanol reactor. This method is like pre-treatment of the syngas to condition the feed to the methanol loop. The drawback of this method is that it requires minimum two unit operation, water gas shift and $CO_2$ removal before the methanol loop, which adds to the number and cost of the equipment and the complexity of running the process. Further, for a low M value syngas, a significant amount of carbon is lost as $CO_2$, which does not have any fuel value, due to purging.

In FIG. 2 another prior art synthesis system is shown. Here, hydrogen is recovered in a hydrogen recovery unit to preferentially recycle hydrogen gas from the unreacted gas to the reactor. Compared to configuration of FIG. 1, this system does not use pre-conditioning of syngas feed and therefore has less complexity and equipment. This configuration increases the hydrogen fed to the reactor and raises the stoichiometric number. When this configuration is used with fresh syngas lean in hydrogen (i.e., low M value) a significant amount of carbon in the unreacted gas is lost because a large fraction of unreacted gas has to be purged to recover enough hydrogen to increase the loop M value

Both known configurations described in FIGS. 1 and 2 result in higher specific natural gas (NG) consumption for fresh syngas with sub-stoichiometric hydrogen concentration (i.e., low M value) because a significant portion of carbon oxides is lost in the process of boosting the concentration of hydrogen in the methanol reactor feed. Further, the configuration of FIG. 1 requires two additional pre-treatment process equipment and process steps for the syngas. Further, the methods do not directly address the problem of hydrogen loss in the methanol reactor due to formation of water according to Equation (3) above.

Accordingly, improvements are needed to overcome these shortcomings, particularly in methanol synthesis where the syngas used as feed has an initially low stoichiometric or M value.

SUMMARY

A method of synthesizing methanol is accomplished by introducing a reactor feed stream from a feed gas source comprising hydrogen gas ($H_2$) and carbon oxides of CO and $CO_2$ into a methanol synthesis reactor. A reactor product stream is formed in the reactor comprising methanol, water, and unreacted carbon oxides of CO and $CO_2$ and hydrogen gas. Methanol and water are separated from the reactor product stream leaving an unreacted gas stream. At least one water gas shift (WGS) unit is provided for converting CO and water to a WGS product stream of $CO_2$ and hydrogen gas. Operation A and optionally operation B are performed wherein A is introducing at least a portion of fresh reactor feed from the feed gas source into the at least one WGS unit to provide a WGS product stream of $CO_2$ and hydrogen gas and B is introducing at least a portion of the unreacted gas stream and steam into the at least one WGS unit for converting unreacted CO and water to a WGS product stream of $CO_2$ and hydrogen gas, and At least a portion of the WGS product stream from operation A and optionally operation B is introduced into a hydrogen recovery unit for producing a hydrogen-rich stream, at least a portion of the hydrogen-rich stream being recycled and introduced into the methanol synthesis reactor as part of the reactor feed stream.

In particular embodiments, the fresh syngas reactor feed from the feed gas source has an M value of less than 2, while in others the fresh syngas reactor feed from the feed gas source has an M value of from 1.9 or less. In still other instances, the reactor feed stream that is introduced into the methanol synthesis reactor has an M value of from 4 or more.

In certain applications, the entire WGS product stream from the WGS unit is introduced into the hydrogen recovery unit inlet. In some embodiments, both operations A and B are performed. And in some embodiments, at least a portion of the unreacted gas stream is introduced directly into the hydrogen recovery unit without being introduced into a WGS unit. In still other embodiments, at least a portion of the WGS product stream is introduced into the hydrogen recovery unit to form the hydrogen-rich stream and another portion of the WGS product stream is introduced into the methanol synthesis reactor without being introduced into the hydrogen recovery unit to form part of the reactor feed stream.

In particular embodiments, operations A and B are both performed using different WGS units. In others, operations A and B are both performed using the same WGS unit.

The feed gas source includes the product of catalytic partial oxidation of natural gas.

The hydrogen recovery unit may be a pressure swing adsorption unit. In other instances, the hydrogen recovery unit may be a hydrogen separation membrane unit.

A system for the synthesis of methanol is also provided. The system includes a methanol synthesis reactor having a reactor inlet for receiving a reactor feed stream from a feed gas source comprising hydrogen gas ($H_2$) and carbon oxides of CO and $CO_2$. The feed gas source is converted in the reactor to a reactor product comprising methanol, water, and unreacted carbon oxides and hydrogen gas. The reactor has a reactor outlet for discharging a reactor product stream of the reactor product from the reactor. The system further includes a separation unit having a separator inlet in fluid communication with the reactor outlet for receiving the reactor product stream and separating methanol and water from the reactor product stream to leave an unreacted gas stream that is discharged from the separation unit through an unreacted gas outlet. At least one water gas shift (WGS) unit for converting CO and water to a WGS product stream of $CO_2$ and hydrogen gas is provided with the system. The at least one WGS unit has a WGS inlet and a WGS outlet. The system also includes a hydrogen recovery unit having a hydrogen recovery unit inlet that is in fluid communication with at least one of the unreacted gas outlet for receiving at least a portion of WGS product stream from the WGS unit. The hydrogen recovery unit has a hydrogen recycle gas outlet that is in fluid communication with the reactor inlet for discharging a hydrogen-rich stream from the hydrogen recovery unit to the reactor inlet as a hydrogen gas recycle stream. The WGS inlet of the at least one WGS unit is positioned at location A and optionally location B, wherein A is a position upstream of the methanol synthesis reactor wherein at least a portion of the reactor feed stream is delivered to the WGS inlet. The WGS outlet being in fluid communication with the hydrogen recovery unit inlet so that at least a portion of the WGS product stream is delivered to the hydrogen recovery unit. And where B is a position downstream of the methanol synthesis reactor wherein the WGS inlet is in fluid communication with the unreacted gas outlet so that the WGS inlet receives at least a portion of the unreacted gas stream from the unreacted gas outlet. And wherein the WGS outlet is in fluid communication with the hydrogen recovery unit inlet so at least a portion of the WGS product stream from the WGS unit is delivered to the hydrogen recovery unit inlet.

In particular embodiments, the WGS inlet is positioned at B and the WGS outlet delivers all of the WGS product stream from the WGS unit to the hydrogen recovery unit inlet. In other embodiments, the WGS inlet is positioned at B and the WGS outlet is split into first and second outlets with the first outlet being in fluid communication with hydrogen gas recovery unit inlet and the second outlet being in fluid communication with the reactor inlet so that a portion of the WGS product stream is delivered to the hydrogen recovery unit inlet and another portion of the WGS product stream is delivered to the reactor inlet as part of the reactor feed stream. In still other instances, the WGS inlet is positioned at both A and B. In some embodiments the at least one WGS unit is a single WGS unit having a WGS inlet positioned at both A and B.

In particular instances, the WGS outlet of the at least one WGS unit is split into first and second outlets with the first outlet being in fluid communication with hydrogen gas recovery unit inlet and the second outlet being in fluid communication with the reactor inlet so that a portion of the WGS product stream is delivered to the hydrogen recovery unit inlet and another portion of the WGS product stream is delivered to the reactor inlet as part of the reactor feed stream. In some embodiments, the hydrogen recovery unit has a hydrogen recovery unit inlet in fluid communication with the unreacted gas outlet of the separation unit for receiving the unreacted gas stream from the separation unit without being introduced into a WGS unit.

## BRIEF DESCRIPTION OF THE DRAWINGS

For a more complete understanding of the embodiments described herein, and the advantages thereof, reference is now made to the following descriptions taken in conjunction with the accompanying figures, in which:

FIG. 1 is schematic flow diagram of a prior art methanol synthesis system utilizing a water gas shift (WGS) treatment of a syngas feed with carbon dioxide ($CO_2$) rejection to increase the stoichiometric number of the feed to the methanol reactor;

FIG. 2 is a schematic flow diagram of a prior art methanol synthesis system utilizing a hydrogen recovery unit of unreacted syngas without additional processing that increases hydrogen concentration to the reactor to increase the stoichiometric number of the feed;

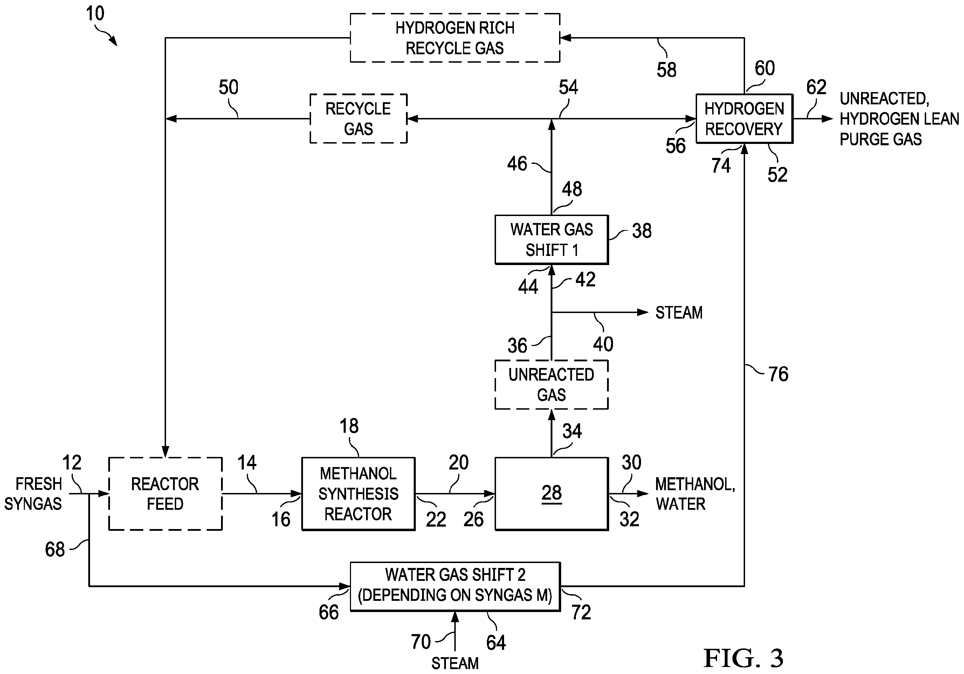

FIG. 3 is a schematic flow diagram of a methanol synthesis system configured in accordance with particular embodiments of the invention that utilizes both a hydrogen recovery unit and WGS treatment of a portion of fresh syngas and the unreacted syngas within the system recycle loop to increase the stoichiometric number of the feed to the methanol reactor;

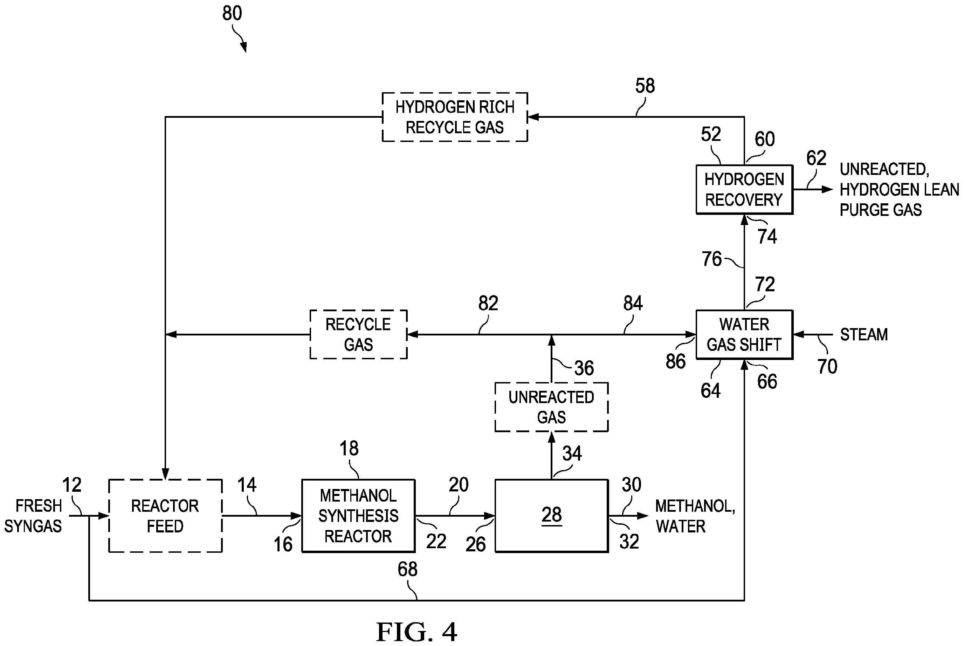

FIG. 4 is a schematic flow diagram of a methanol synthesis system configured in accordance with certain embodiments of the invention that utilizes both a hydrogen recovery unit and a WGS treatment of a portion of the fresh syngas and a portion of the unreacted syngas within the system recycle loop to increase the stoichiometric number of the feed to the methanol reactor, with a portion of the unreacted syngas being recycled to the methanol reactor without any WGS treatment; and

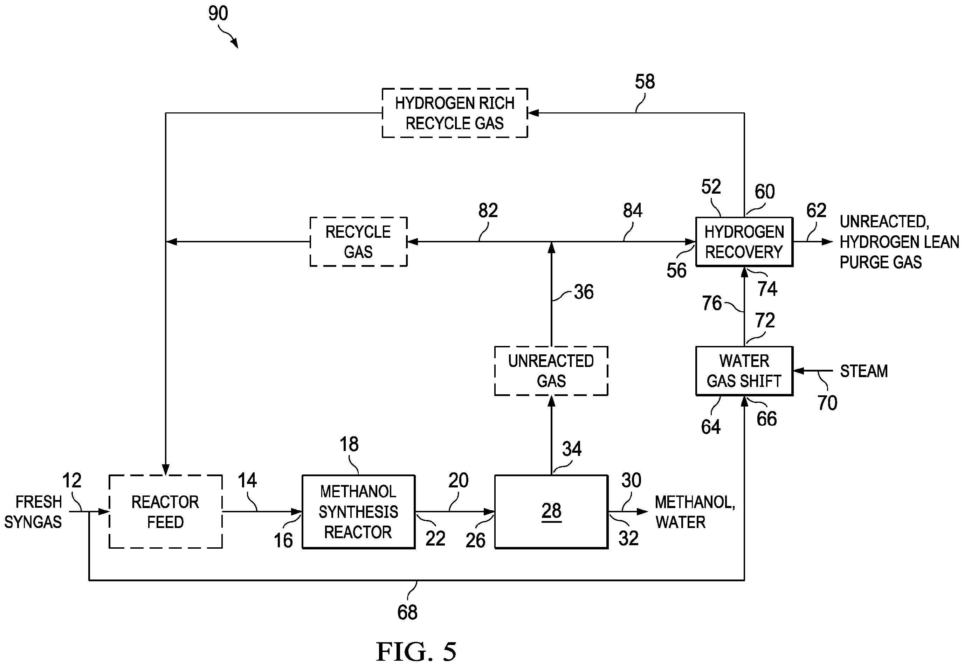

FIG. 5 is a schematic flow diagram of a methanol synthesis system configured in accordance with certain embodiments of the invention that utilizes both a hydrogen recovery unit and a WGS treatment of a portion of the fresh syngas, with a portion of the unreacted syngas being recycled to the methanol reactor without any WGS treatment and another portion being fed directly to the hydrogen recovery unit without any WGS treatment.

## DETAILED DESCRIPTION

One-way to reduce the purge gas fraction (and increase conversion) in methanol synthesis reactions of syngas is by increasing the hydrogen concentration in the feed to the reactor. This can be achieved by increasing the hydrogen concentration in either a portion of the fresh syngas or the unreacted gas after methanol conversion and recovering the hydrogen with suitable methods. Using a water gas shift (WGS) reactor that carries out WGS reaction to convert some of the CO in the unreacted product gas or the fresh syngas feed to hydrogen can increase the relative concentration of hydrogen. The WGS reaction is illustrated by Equation (4) below:

$$WGS = CO + H_2O \rightarrow CO_2 + H_2 \qquad (4)$$

This reaction can directly counter the impact of hydrogen loss to water during methanol synthesis through reaction in Equation (3). The water is added back as steam and the WGS reaction converts it back to hydrogen thus offsetting the water formation in methanol reactor.

Sending this converted gas stream from the WGS reaction for hydrogen recovery through a pressure shift adsorption (PSA) unit or membrane separator and sending the recovered hydrogen as feed to the methanol reactor builds up hydrogen in the reactor feed at a lower purge fraction. This allows higher carbon conversion while maintaining the required stoichiometric number in the feed to the reactor. FIG. 3 illustrates a system 10 for methanol synthesis that utilizes WGS reaction for additional hydrogen recovery in a recycle loop.

As shown in FIG. 3, a fresh syngas feed 12 of the system 10 is introduced as part of the reactor feed 14 into the inlet 16 of a methanol synthesis reactor 18. The fresh syngas 12 is from a feed gas source (not shown) and comprises hydrogen gas ($H_2$) and carbon oxides of CO and $CO_2$. The relative amounts of these different components may vary in the feed gas. The fresh syngas may undergo pretreatment to remove harmful or undesirable compounds prior to use. The feed gas source in the embodiments and systems that are described herein may be those that typically provide a fresh feed gas having a low M value according to Equation (1) of from 2, 1.9, 1.85, 1.8. 1.75, 1.7, 1.65, 1.6, 1.55, 1.5 or less and a $H_2$/CO molar ratio of less than 3. Such fresh feed gases typically having such low M values may be those derived from the catalytic partial oxidation, non-catalytic partial oxidation or the autothermal reforming (ATR) of hydrocarbons, such as natural gas. Feed gases from other processes may also be used, such as those from combined reforming, reforming of heavier hydrocarbons, gasification of carbon sources like coal, biomass, and/or municipal solid waste. While in many embodiments, the fresh feed gas may have a low M value of from 2 or less, in others the M value of the fresh feed gas may have an M value of more than 2, more particularly from 2.05, 2.1, 2.15, 2.2 or more.

It should be noted in the description, if a numerical value, concentration or range is presented, each numerical value should be read once as modified by the term "about" (unless already expressly so modified), and then read again as not so modified unless otherwise indicated in context. Also, in the description, it should be understood that an amount range listed or described as being useful, suitable, or the like, is intended that any and every value within the range, including the end points, is to be considered as having been stated. For example, "a range of from 1 to 10" is to be read as indicating each and every possible number along the continuum between about 1 and about 10. Thus, even if specific points within the range, or even no point within the range, are explicitly identified or referred to, it is to be understood that the inventor appreciates and understands that any and all points within the range are to be considered to have been specified, and that inventor possesses the entire range and all points within the range.

The methanol synthesis reactor 18 may be that conventionally known and is operated at conditions suitable for methanol synthesis. Such methanol reactors will typically contain a methanol synthesis catalyst, such as a copper catalyst in combination with other metal oxides, such as zinc, aluminum, chromium, titanium, zirconium, magnesium, silicon etc. Typical reaction pressures may range from 4,000 kPa to 15,000 kPa, more particularly from 6,000 kPa to 10,000 kPa. Operating temperatures may range from 160 °C to 350 °C, more particularly from 200 °C to 300 °C, and still more particularly form 210 °C to 280 °C. The size and configuration of the reactor 18 may be constrained by the maximum temperature of the catalyst used and to decrease or limit the formation of higher alcohols.

The methanol synthesis in the reactor 18 forms a reactor product stream 20 comprising methanol, water, and unreacted gases of carbon oxides (CO and $CO_2$) and hydrogen gas. The reactor product stream 20 is discharged from the reactor 18 through reactor outlet 22. The composition of the reactor product stream 20 will typically be composed of from 5 mol% to 25 mol% of methanol by total moles of the product stream, from 50 mol% to 85 mol% hydrogen by total moles of the product stream, from 1 mol% to 10 mol% CO by total moles of the product stream, and from 1 mol% to 7 mol% $CO_2$ by total moles of the product stream. Typical conversions across the methanol reactor may range from 10 mol% to 30 mol% $CO_2$ conversion, from 30 mol% to 90 mol% CO conversion and from 10 mol% to 40 mol% $H_2$ conversion. These conversions are based on moles of the species in the feed to and product from the methanol reactor.

The reactor outlet 22 is in fluid communication with the inlet 26 of a separation unit 28 that receives the reaction product stream 20. Although not shown, and as will be understood by those skilled in the art, the product stream 20 is typically cooled by use of a heat exchanger (not shown) prior to introduction into the separation unit 28 to condense liquids in the product stream 20. The separation unit 28 separates liquid methanol and water from the reactor product stream 20 to form a methanol product stream 30 that is discharged from the separator 28 through separator product outlet 32 for collection, storage, and/or further processing. The separated unreacted gases are discharged from the separation unit 28 through an unreacted gas outlet 34 as unreacted gas stream 36.

For ease of description, certain secondary process equipment, such as compressors, pumps, separators, heat exchangers, chillers, conduits, collection vessels, etc., of the systems described herein may not have not been shown or discussed. It should be apparent to those skilled in the art, however, that such secondary process equipment associated with the process equipment described or shown herein may be a part of the systems as well. Accordingly, while such secondary process equipment is not shown or described, any such equipment necessary for carrying out the process steps for the systems described herein will be understood to have been included. Additionally, while in the description and claims it is described that certain items are in communication or interrelated with one another, there may be intervening secondary process equipment (e.g., heat exchangers, compressors, etc.) so that the various process equipment may not be in direct communication with one another but may communicate through such intervening secondary process equipment. Thus, when it is stated that the process equipment of the various systems are in communication or otherwise interrelated with one another in a certain manner it should be construed that these may be in communication or interrelated directly to one another or indirectly through such necessary secondary equipment that has not necessarily been shown or described.

In the embodiment of FIG. 3, the unreacted gas stream 36 is introduced into a water gas shift (WGS) reactor unit 38 along with a cofeed of steam 40. The unreacted gas stream 36 is typically heated prior to introduction into the WGS unit 38. The steam cofeed 40 can provide all or part of this heating or the unreacted gas stream 36 may be heated separately through a separate heat exchanger (not shown). Process temperatures for the WGS unit 38 typically range from 250 °C to 400°C. Pressures within the WGS unit 38 will typically range from 5,000 kPa to 10,000 kPa and may be the same range or similar to the operating pressures of the methanol synthesis reactor 18 or "loop" pressures of the reactor system 10. The unreacted gases 36 and steam may be pressurized or expanded to provide the necessary pressures for the WGS operation. While the unreacted gas 36 and steam 40 are shown as being introduced as a combined feed 42, in other embodiments the streams 36 and 40 may be introduced separately into the WGS unit 38. The combined feed 42 or separate feeds of unreacted gases 36 and steam 40 are introduced through a WGS inlet 44 located downstream of the reactor 18 and into the WGS unit 38 where they are contacted with a suitable WGS catalyst contained within the unit 38. A non-limiting example of a suitable WGS catalyst includes cobalt molybdenum sulfide. The WGS catalyst is that suitable at the operating pressures of the WGS unit 38, which may be different from WGS catalysts that are used treating the syngas feed, as will be discussed later on.

In the WGS reactor, the unreacted CO and water from the steam are converted to $CO_2$ and $H_2$ according to Equation (4), previously discussed. The produced $CO_2$ and $H_2$, along with any remaining unreacted gases, are discharged from the WGS unit 38 as WGS product stream 46 through a WGS outlet 48. The targeted CO to hydrogen conversion in the WGS reactor is decided based on the desired M value at methanol reactor inlet for a given composition of fresh syngas and the methanol loop configuration and operating conditions, For example, to obtain an M value above 5 at the methanol reactor inlet, the CO to $H_2$ conversion in the WGS reactor is typically maintained between 30 mol% to 100 mol% but with the presence of a WGS treatment unit 64 for fresh syngas, the typical CO conversion is maintained between 0-30 mol%. These reported conversion in WGS reactor values are indicative to demonstrate an operating window and can be optimized depending on the methanol reactor size and operating parameters and the fraction of gas flow to the hydrogen recovery unit. The product stream 46 may be cooled by exchanging heat with the feed stream 42 or 36 to a temperature typically below 45 °C after being discharged from the WGS unit 38. The cooled product stream 46 may be routed through a vapor liquid separator (not shown) to remove any condensed liquids. In some instance, streams 50 and 54 may be cooled to a different temperature, with stream 54 being hotter than stream 50 when pressure swing adsorption (PSA) is used for hydrogen recovery and the PSA is operating at a pressure lower than WGS unit 38. Suitable heat exchange or cooling equipment (not shown) to recover this heat for heating another cold stream may be provided for this purpose. The composition of the WGS product stream 46 will depend on the CO conversion in the WGS unit 38. The CO conversion will typically range from 0-100 mol% depending on the desired M value at the methanol reactor inlet and operating parameters and design of the methanol reactor. As shown in FIG. 1, the WGS product stream 44 may be split wherein a portion of the WGS product stream 44 is recycled as a recycle stream 50 that forms part of the recycle loop that is introduced into the methanol synthesis reactor 18 as part of the reactor feed 14.

A portion of the WGS product stream 46 is also split as a WGS product stream 54 and delivered after cooling and pressure adjustment (expansion in cases of PSA and compression or no pressure change in case of membrane separation) to a hydrogen recovery unit 52 through hydrogen recovery unit inlet 56, which is in fluid communication with the WGS outlet 48. The WGS product stream 54 contains unreacted gases from the reactor 18 and thus constitutes an unreacted gas stream. The amount of WGS product stream 46 that is delivered to the hydrogen recovery unit 52 as WGS product stream 54 may range from 1 mol% to 99 mol% by total moles of the WGS product stream 46, more particularly from 10 mol% to 75 mol% by total moles of the WGS product stream 46, and still more particularly from 10mol% to 60 mol% by total moles of the WGS product stream 46, with the remainder being recycled to the reactor 18 as the recycle stream 50. If WGS unit 64 is present for converting a portion of the fresh syngas feed to hydrogen that is then delivered to the hydrogen recovery unit 52, then the amount of WGS product stream 46 that is delivered to the hydrogen recovery unit 52 as WGS product stream 54 may range from 1 mol% to 20 mol% because from the hydrogen from the additional WGS unit delivers additional hydrogen rich feed for the hydrogen recovery unit 52.

The hydrogen recovery unit 52 may be a PSA unit and/or hydrogen membrane separation unit for producing a hydrogen-rich stream containing higher concentrations of $H_2$ gas. PSA units may operate at lower pressures than the loop or methanol synthesis reactor pressures. Such pressures may range from 2,500 kPa to 4,000 kPa. The WGS product stream may require expansion and heating to reach the pressure and temperature for the PSA. Typical recovery of hydrogen gas through PSA units may range from 50 mol% to 90 mol% by total moles of hydrogen gas of the hydrogen-gas-containing feed, more particularly from 80 mol% to 90 mol% by total moles of hydrogen gas of the hydrogen-gas-containing feed.

Membrane separation for hydrogen gas may also be used for producing a hydrogen-rich stream containing higher concentrations of $H_2$ gas. Such membrane separation units may accept feed at pressures similar to the methanol synthesis reactor or loop. Typical recovery of hydrogen gas from the membrane separation units may range from 60 mol% to 98 mol% by total moles of hydrogen gas of the hydrogen-gas-containing feed, more particularly from 80 mol% to 95 mol% by total moles of hydrogen gas of the hydrogen-gas-containing feed. Recovery of $CO_2$ may range from 1 mol% to 15 mol% by total moles of $CO_2$ gas in the hydrogen-gas-containing feed. Minor amounts of other gases, such as methane, nitrogen and CO, may also be recovered.

In certain instances, a combination of PSA and membrane separation units may be used as the hydrogen recovery unit 52. A combination of PSA and membrane units allows the precise control of $H_2$/$CO_2$ ratio in the recovered hydrogen rich stream 58 to be achieved.

The hydrogen recovery unit 52 discharges a hydrogen-rich stream 58 from the hydrogen recovery unit 52 through a hydrogen recycle gas outlet 60 that is in fluid communication with the reactor inlet 16. The hydrogen-rich stream 58 is delivered as a hydrogen gas recycle stream as part of the reactor feed stream 14 to the reactor 18. The hydrogen-rich recycle stream 58 may have a hydrogen gas content of from 60 mol% to 99 mol% by total moles of the hydrogen-rich recycle stream, more particularly from 80 mol% to 99 mol% by total moles of the hydrogen-rich recycle stream, and still more particularly from 90 mol% to 99 mol% by total moles of the hydrogen-rich recycle stream. The remaining constituents of stream 58 will typically contain other components present in the unreacted gas like CO, $CO_2$, water, methanol, methane, nitrogen. The composition of other constituents depends on the hydrogen recovery method. The hydrogen rich stream 58 may be compressed to increase its pressure to satisfy the requirements of operating pressure of the methanol reactor. The gas compression may be done either before or after mixing with fresh feed stream 12 depending on the pressure of the fresh feed stream 12.

The remainder of the gases discharged from the hydrogen recovery unit 52 is discharged as a hydrogen-lean gas stream that is purged from the hydrogen recovery unit 52 as purge gas stream 62. The purge gas stream 62 will typically contain unreacted CO, $CO_2$, methane, nitrogen and $H_2$ gases. Typically the purge gas composition will be such that it will have calorific value in the range of 0-10 MMBtu/ton methanol, particularly in the range of 1-5 MMBtu/to methanol. It can be used as fuel source for other energy requirements in the methanol system 10 or elsewhere.

The hydrogen-rich stream 58, the WGS product recycle stream 50, and fresh syngas feed 12 may be combined or introduced separately after required pressure adjustment by compression to form the reactor feed 14. The hydrogen-rich stream 58 will typically make up from 5 mol% to 25 mol% by total moles of the reactor feed 14. The WGS product recycle stream 50 will typically make up from 50 mol% to 80mol% by total moles of the reactor feed 14. And the fresh syngas feed 12 will typically make up from 10 mol% to 30 mol% by total mole of the reactor feed 14.

Because the stream 58 is rich in hydrogen gas, this can be used to raise the M value of the feed from that supplied by the fresh syngas feed 12. In particular, enough hydrogen gas can be recycled in the system 10 or within the loop to provide an M value for the reactor feed 14 of from 4 or more, with an M value of from 5 to 5.5 being useful for most methanol synthesis reactors. Thus, even with fresh syngas feed having low M values of from 2 or less, the system 10 with a recycle loop employing the WGS and hydrogen recovery units within the loop provides a higher M value for the feed and reduces the purge fraction that is discharged from the system to achieve that higher M value.

This is in contrast with prior art systems that typically operate by either conditioning the fresh syngas using at least two additional unit operation of $CO_2$ rejection and water gas shift or purging larger fractions of unreacted gases and without any WGS within any recycle loop. In such systems, a syngas feed lean in hydrogen results in a much lower hydrogen concentration in the unreacted gas in the product stream. As a result, a large purge fraction is required to recover enough hydrogen to achieve a targeted M value in the reactor feed. This results in a loss of unreacted carbon oxides, which in turn reduces the carbon conversion in the loop.

In another benefit of the configuration, the formation of hydrocarbons like waxes in the methanol reactor can be reduced due to the presence of higher hydrogen in the feed to the methanol reactor. Higher hydrogen concentration will promote the reaction of carbon oxides with hydrogen over the reaction of carbon-carbon bond formation. This benefit is also applicable for fresh syngas with M value of more than 2.0, in the range of from 2 to 2.1.

FIG. 3 also shows a variation wherein a second WGS unit 64 is provided with the system 10. In other embodiments the second WGS unit 64 may not be included. In still other embodiments, the WGS unit 64 may be used and the WGS unit 38 may be eliminated. The WGS unit 64 has a WGS inlet 66 that is positioned upstream of the methanol synthesis reactor 18 and receives a portion of the fresh syngas 12 of the reactor feed 14 as a slip or side stream 68. The slip stream may be 0-50 mol% of the fresh syngas stream 12 and more particularly from 2 mol% to 20 mol% of the fresh syngas stream 12. The slip or side stream 68 may be adjusted in temperature and pressure for treatment in the WGS unit 64, if necessary, by the use of heat exchange and compression equipment (not shown). A steam feed 70 is also fed to the WGS unit 64 as a combined or separate feed from the syngas side stream 68. The fresh syngas of the side stream 68 is contacted with a suitable WGS catalyst within the WGS unit 64. Typically, the WGS unit 64 is operated with the CO to $H_2$ conversion ranging from 0-100 mol%, more particularly from 10 mol% to 95 mol %. The WGS unit 64 may operate at lower operating pressures (e.g., 2,000 kPa to 3,500 kPa) than the WGS unit 38 within the recycle loop and may employ a different catalyst. A non-limiting example of a suitable WGS catalyst for the WGS unit 64 may include copper zinc aluminum. These may be WGS catalysts that are suitable at the operating pressures of the WGS unit 64.

The WGS unit 64 converts CO and water from the syngas 68 and steam feed 70 to $CO_2$ and hydrogen gas,

according to Equation (4). An outlet 72 of the WGS unit 64 is in fluid communication with the an inlet 74 of the hydrogen recovery unit 52, with any necessary cooling, as previously described, and pressure adjustment (expansion or compression) for delivering a WGS product stream 76 to the inlet 74 and into the hydrogen recovery unit 52. Alternatively, the WGS product stream 76 can be combined with the WGS product stream 54 and delivered to inlet 56 after any required pressure adjustments. This additional WGS product stream further increases the amount of hydrogen gas that may be recovered in the hydrogen recovery unit 52 and which is recycled as hydrogen-rich recycle stream 58. The composition of the WGS product stream 76 will typically be composed of from 60 mol% to 80 mol% $H_2$ by total moles of the WGS product stream, from 10 mol% to 30 mol% CO by total moles of the WGS product stream, and from 5 mol% to 20 mol% $CO_2$ by total moles of the WGS product stream. Remaining in the product stream 76 is methane, and inert gases, like nitrogen and steam.

The amount of fresh syngas diverted to the WGS unit 64 may vary depending upon the fresh syngas composition. The fraction of fresh syngas stream 68 diverted to WGS unit 64 and the CO to $H_2$ conversion the WGS unit 64 varies depends on the desired M value at the methanol inlet and the composition of the fresh syngas. For a syngas that has high M value, a smaller fraction is diverted to the WGS unit 64 because of higher hydrogen content in the syngas. At a still higher M value of syngas the hydrogen content in the syngas is high enough to directly send the slip stream 68 from fresh syngas to the hydrogen recovery unit without the need for the WGS unit 64. Using the WGS unit 64, however, will reduce the fraction of syngas sent to hydrogen recovery unit. For instance, when the fresh syngas has an M value of 1.85, a slip stream fraction of from 8 mol% to 9 mol% without any WGS unit 64 (zero CO conversion) that is sent directly to the hydrogen recovery unit 74 is required to achieve an M value of 5.3 at the methanol reactor inlet. This is in contrast to a slip fraction that is reduced to from 5 mol% to 6 mol% when the WGS unit 64 is used and operated at 90% conversion to achieve the same M value. On the other hand, when the fresh syngas has an M value of 1.75, then 9 mol% to 10 mol % slip stream with a WGS unit, such as the unit 64, operating at 90 mol% CO conversion is required to achieve 5.3 M value at methanol reactor inlet. The slip stream fraction increases to from 12 mol% to 13 mol% if the WGS unit 64 is operated at a lower CO conversion of from 30 mol% to 32 mol%. The CO conversion in WGS unit 64 and the amount of fresh syngas delivered as stream 68 to the WGS inlet 66 may depend upon the M value of the fresh syngas feed.

Additionally, in some embodiments, where the M values are slightly higher (e.g., 1.85 or more), both the WGS unit 64 and 38 may be eliminated with the slip or side stream 68 of the fresh syngas 12 being fed directly to the inlet 74 of the hydrogen recovery unit 52 without the use of the WGS unit or need for an additional WGS product stream. Additionally in some embodiments, where the M values are around 1.78 or lower the WGS unit 38 may be eliminated and only WGS unit 64 may be used with a slip or side stream 68 of fresh syngas 12 and product from WGS unit 64 be fed into the hydrogen recovery unit 52. This may provide sufficient hydrogen for recovery and recycle to raise the M value of the reactor feed 14 to 5 or more.

Referring to FIG. 4, another variation of a methanol synthesis system 80 is shown. The system 80 is similar to the system 10 previously described with similar components labeled with the same reference numerals. Additionally, the configuration and operating conditions of the various components may be the same or similar unless otherwise stated.

The system 80 differs from system 10 of FIG. 3 in that a portion of the unreacted gas stream 36 from the unreacted gas outlet 34 of the separator 28 is split off into a recycle gas stream 82 that is introduced into the methanol synthesis reactor 18 as part of the recycle loop to the reactor feed 14 without undergoing any treatment through a WGS unit.

The remaining portion of the unreacted gas stream 36 can be directed as stream 84 to WGS inlet 86 of WGS unit 64, which may be same or similar to WGS unit 64 of system 10. The stream 84 may be heated and expanded to reach the operating pressure and temperature of WGS unit 64. This may be a particularly useful configuration because it uses only one WGS reactor for both the fresh syngas and unreacted purge gas. Although the WGS unit 64 is shown with two separate inlets 66 (i.e., an upstream inlet) and inlet 86 (i.e., a downstream inlet), in certain embodiments, the stream 84 may be combined with the stream 68 and introduced as a combined stream into a single inlet. In such cases, the single inlet would constitute both an upstream inlet and a downstream inlet as the inlet is receiving both upstream and downstream fluid flow. All of the WGS product stream 76 from the WGS unit 72 is delivered from WGS outlet 72 to the hydrogen recovery unit inlet 74 and to the hydrogen recovery unit 52.

The amount of the unreacted gas stream 36 that is delivered as unreacted gas feed 84 to the WGS unit 64 in system 80 may range from 1 mol% to 99 mol% by total moles of the unreacted gas stream 36, more particularly from 5 mol% to 60 mol% by total moles of the unreacted gas stream 36, and still more particularly from 7 mol% to 30 mol% by total moles of the unreacted gas stream 36, with the remainder being recycled to the reactor 18 as recycle gas stream 82.

The slip stream 68 may be 0-50 mol% of the fresh syngas stream 12 and more particularly from 2 mol% to 20 mol% of the fresh syngas stream 12. The slip or side stream 68 may be adjusted in temperature and pressure for treatment in the WGS unit 64, if necessary, by the use of heat exchange and compression equipment (not shown). While using this option, the amount of the unreacted gas stream 36 that is delivered as unreacted gas feed 84 to the WGS unit 64 in system 80 may range from 1 mol% to 20 mol% by total moles of the unreacted gas stream 36.

The hydrogen-rich recycle stream 58 will typically make up from 2 mol% to 20 mol% by total moles of the total reactor feed 14. The unreacted gas recycle stream 82 will typically make up from 50 mol% to 80 mol% by total moles of the total reactor feed 14. And the fresh syngas feed 12 will typically make up from 10 mol% to 40 mol% by total moles of the total reactor feed 14.

Referring to FIG. 5, another embodiment of a methanol synthesis system 90 is shown. The system 90 is similar to the systems 10 and 80 previously described, with similar components labeled with the same reference numerals. Additionally, the configuration and operating conditions of the various components may be the same or similar unless otherwise stated.

In the system 90, only the fresh feed gas side stream 68 is delivered to WGS unit 64, with the stream 84 of the unreacted gas stream 36 being directed directly to the hydrogen recovery unit inlet 56 of the hydrogen recovery unit 52 without undergoing any treatment in a WGS unit. The slip stream 68 may be composed of from 0-50 mol% of the fresh syngas stream 12, more particularly from 2 mol% to 20 mol% of the fresh syngas stream 12. The slip or side stream 68 may be adjusted in temperature and pressure for treatment in the WGS unit 64, if necessary, by the use of heat exchange and compression equipment (not shown). While using this option, the amount of the unreacted gas stream 36 that is delivered as unreacted gas feed 84 directly to hydrogen recovery unit 52 may range from 1 mol% to 20 mol% by total moles of the unreacted gas stream 36.

As can be seen from the various systems described above, it is possible to use syngas for synthesizing methanol that is low in hydrogen, low or high in CO, and/or low or high in $CO_2$ concentration by making use of hydrogen recovery within a recycle loop of the system in combination with water gas shift treatment, with or without $CO_2$ rejection, and/or reverse water gas shift treatment. Modifications to the various systems described above can also be made to achieve the desired methanol conversion with optimal use of carbon. The typical value ranges can also change based on process conditions used.

The methanol synthesis systems described herein allow utilization of lower amounts of natural gas for making a wide variety of syngas compositions for the same conversion of carbon to methanol.

The following examples serve to further illustrate various embodiments and applications

EXAMPLES

EXAMPLE 1

Computer simulations were run for methanol conversion of syngas utilizing a methanol synthesis system like that of system 10 of FIG. 3, with a WGS unit like the WGS unit 38 in the methanol loop for converting unreacted gases and a side stream WGS treatment of the syngas feed gases with WGS unit like the unit 64. A pressure swing adsorption (PSA) unit was used for hydrogen recovery. For comparison purposes, simulations were also conducted on conventional methanol conversion systems configured as shown in FIGS. 1 and 2. All process configurations were simulated using ASPEN Plus process simulation software provided by Aspen Technology Inc. The simulations used PSRK equation of state to model the thermodynamic behavior of the molecules.

The methanol reactor was modeled using an equilibrium reactor with the two reactions shown in Equations (2) and (3). The equilibrium model calculates the equilibrium conversions of reactants for a specified temperature and pressure and an inlet gas composition. The water gas shift reactors are modeled as stoichiometric reactors with specified conversion for CO to $CO_2$. The WGS reactor is assumed to be adiabatic. The various separation unit-operations like hydrogen recovery using PSA or membrane and $CO_2$ rejection are modeled using component splitter blocks in Aspen Plus. The operating conditions and the separation efficiency are specified for these blocks based on available data about these unit operations.

The input composition of fresh syngas to the methanol loop is based on composition obtained from syngas technologies like catalytic partial oxidation or auto thermal reforming. The typical syngas composition for a fresh syngas with M value of 1.85 is 63.5 mol% $H_2$, 26.7 mol% CO, 5.0 mol% $CO_2$, 0.4 mol% $H_2O$, with the remainder being inerts like methane and nitrogen. This represents the typical composition obtained when natural gas is converted to syngas under typical operating conditions of autothermal reforming. The typical syngas composition for a fresh syngas with M value of 1.75 is 64.3 mol% $H_2$, 29.8 mol% CO, 4.4 mol% $CO_2$, 0.4 mol% $H_2O$, with the remainder being inerts like methane and nitrogen. This represents the typical composition obtained when natural gas is converted to syngas under typical operating conditions of catalytic partial oxidation. A PSA operating at 3,500 kPa and 45 °C with a separation efficiency of 85 mol% for hydrogen, 5 mol% for CO and 3 mol% for $N_2$ is used as the hydrogen recovery unit.

The feed to the PSA is first heated to 100 °C and then expanded in a turbine to reach the operating conditions of the PSA unit. The WGS unit in the loop (i.e., WGS unit 38) is operated at pressure similar to the methanol reactor outlet pressure. The feed temperature and conversion of CO to $CO_2$ is presented in Table 1 below. The feed to the WGS reactor is heated to the temperature mentioned in the table and the product from the WGS reactor is cooled to 45 °C by exchanging heat with reactant feed gases. The WGS unit for the fresh syngas (i.e., WGS unit 64) is operated at a lower pressure of 3,000 kPa with feed temperature and CO conversion presented in Table 1 below. The fraction of fresh syngas sent to the WGS 2 is also presented in Table 1. The same operating conditions are used for operating the WGS unit of the comparative example of FIG 1. For $CO_2$ rejection in FIG. 1, a membrane unit is used with $CO_2$ recovery mentioned in Table 1. The optimal operating conditions for the methanol reactor are listed in Table 1. The feed to the methanol reactor is heated to 165 °C and the product from the methanol reactor is cooled to 45 °C to separate methanol and water from unconverted gas by condensation in a vapor-liquid separator.

The hydrogen recovered from the PSA is mixed with the fresh syngas feed and is compressed to the operating pressure of the methanol reactor before mixing with the recycled fraction of unconverted gas from the methanol reactor. Intermediate cooling and water removal is done during compression of the mixed fresh syngas and recycled hydrogen rich gas. The recycle gas is also compressed to reach the methanol reactor pressure. The results are presented in Table 1 below:

Table 1

| | Fresh Syngas, M = 1.85 | | | Fresh Syngas, M = 1.75 | | |
|---|---|---|---|---|---|---|
| | Comparative | | Embodiment | Comparative | | Embodiment |
| | FIG. 1 | FIG. 2 | FIG. 3 | FIG. 1 | FIG. 2 | FIG. 3 |
| Loop M Value Reactor Feed | 5.3 | 5.3 | 5.3 | 5.3 | 5.3 | 5.3 |
| Purge Fraction (mol%) | 3.2 | 10.8 | 8.0 | 2.8 | 21.5 | 7.0 |
| Carbon Conversion (mol%) | 77 | 76 | 80 | 85 | 73 | 86 |
| Natural Gas (MMBtu/ton MeOH | 33.7 | 34.4 | 32.4 | 30.7 | 35.7 | 30.4 |
| Fresh SG to WGS-2 (mol%) | 100.0 | NA | 4.2 | 100.0 | NA | 8.1 |
| WGS 2 Feed Temperature (C) | 250 | NA | 249 | 250 | NA | 250 |
| WGS 2 CO conversion (mol%) | 5.0 | NA | 90.0 | 2.5 | NA | 90.0 |
| CO2 Rejection (mol%) | 50.0 | NA | NA | 80.0 | NA | NA |
| Methanol Reactor exit T (C) | 210 | 250 | 210 | 210 | 250 | 210 |
| Methanol Reactor P (bar) | 75 | 75 | 75 | 65 | 65 | 65 |
| WGS 1 Feed Temperature (C) | NA | NA | 300 | NA | NA | 300 |
| WGS 1 CO conversion (mol%) | NA | NA | 5.0 | NA | NA | 15.0 |

With the inclusion of the WGS treatment within the methanol loop, the fraction of purge required for hydrogen recovery is reduced (i.e., the system of FIG. 2 versus FIG. 3). This allows more carbon oxides to be recycled back to the reactor and achieve a higher carbon conversion. This is true even while maintaining the desired overall or loop M value for the reactor feed fed to the methanol reactor. The system of FIG. 3 also provided better carbon conversion compared to the system of FIG. 1, where the syngas feed was conditioned using WGS treatment followed by $CO_2$ rejection. As a result of the proposed configuration, significant reduction in the amount of natural gas consumption used to make syngas can be achieved. This is even more significant when the syngas feed has low M value. Additionally, the proposed configuration allows reduction in operating temperature of the methanol reactor. This reduction is more significant in the case of lower M value.

Using the configuration in FIG. 3, in the configuration either without WGS unit 38 or without WGS unit 64, for the fresh syngas with M value of 1.75 and 1.85 also results in 1 - 5 MMBtu/t MeOH reduction in NG consumption as compared to configuration of FIG. 2, which does not use any WGS treatment in the methanol loop. The reduction in NG consumption is higher when syngas feed has low M value.

EXAMPLE 2

Computer simulations using the same simulation software as Example 1 were run for the methanol conversion of syngas utilizing a methanol synthesis system like that of system 80 of FIG. 4. The FIG. 4 system can be operated in two sub- configurations: a) sending only a portion of the unreacted stream to WGS unit 64 and sending all the fresh syngas stream to reactor feed, and b) sending both a portion of unreacted gas stream and a portion of fresh syngas to the WGS unit 64. Both of these configurations were evaluated using the simulation conditions mentioned in Example 1. PSA was used for hydrogen recovery in the loop. For comparison purposes, simulations were also conducted on conventional methanol conversion systems configured as shown in FIG. 1 and FIG. 2. The simulations assumptions and conditions are similar to that mentioned in Example 1.

The WGS reactor is operated at a lower pressure of 3,000 kPa at the feed temperature and conversion mentioned in the table below. The fraction of fresh syngas feed to the WGS reactor is mentioned in the Table 2 below. The WGS feed is heated by exchanging heat with the hot WGS product stream. The pressure of the unreacted gas stream is reduced using a turbine to match the operating pressure of the WGS reactor. The results are presented in Table 2 below;

Table 2

| | Fresh Syngas, M = 1.85 | | | | Fresh Syngas, M = 1.75 | | | |
|---|---|---|---|---|---|---|---|---|
| | Comparative | | Embodiment | | Comparative | | Embodiment | |
| | FIG. 1 | FIG. 2 | FIG. 3 option (a) | FIG. 3 option (b) | FIG. 1 | FIG. 2 | FIG. 3 option (a) | FIG. 3 option (b) |
| Loop M Value Reactor Feed | 5.3 | 5.3 | 5.3 | 5.3 | 5.3 | 5.3 | 5.3 | 5.3 |
| Purge Fraction (mol%) | 3.2 | 10.8 | 8.7 | 8.7 | 2.8 | 21.5 | 16.8 | 8.0 |
| Carbon Conversion (mol%) | 77 | 76 | 80 | 81 | 85 | 73 | 81 | 86 |
| Natural Gas (MMBtu/ton MeOH | 33.7 | 34.4 | 32.8 | 32.3 | 30.7 | 35.7 | 32.2 | 30.4 |
| Fresh SG to WGS (mol%) | 100.0 | NA | 0.0 | 3.8 | 100.0 | NA | 0.0 | 7.9 |
| WGS 2 Feed Temperature (C) | 250 | NA | 250 | 250 | 250 | NA | 250 | 250 |
| WGS CO conversion (mol%) | 5.0 | NA | 90.0 | 90.0 | 2.5 | NA | 90.0 | 90.0 |
| CO2 Rejection (mol%) | 50.0 | NA | NA | NA | 80.0 | NA | NA | NA |
| Methanol Reactor exit T (C) | 210 | 250 | 240 | 210 | 210 | 250 | 240 | 210 |
| Methanol Reactor P (bar) | 75 | 75 | 75 | 75 | 65 | 65 | 65 | 65 |

The use of a WGS unit either to treat a portion of unreacted gas (option (a)) or to treat a portion of unreacted gas and portion of fresh syngas (option (b) allows higher hydrogen recovery at a lower purge value compared to configuration of FIG. 2. This shows that the proposed configurations lead to reduction in NG consumption in comparison to the conventional configuration of FIG. 1 and FIG. 2 due to higher carbon conversion to methanol at the same loop M value in the feed to the methanol reactor. Further, it reduces the operating temperature of the methanol reactor, which can improve catalyst stability. The system of FIG. 4 operated with option (b) had lower NG consumption compared to the configuration of FIG. 1 at similar operating temperature. Further, the system of FIG. 4 with either the option (a) or option (b) modification is a simplified configuration as compared to the conventional configuration of FIG. 1 because the removal of an additional unit operation of $CO_2$ removal from the configuration

Between option (a) and option (b) modification of FIG. 4, the option (b) provided the best results preferred with respect to lower NG consumption.

EXAMPLE 3

A similar comparison of the configuration in FIG. 5 was also made, where a portion of the fresh syngas is send to the WGS unit 64 and the portion of unreacted gas is directly sent to hydrogen recovery feed without any WGS treatment. The configurations shows a lower NG consumption of 32.4 MMBtu/t MeOH and 30.6 MMBtu/t MeOH for fresh syngas of M value 1.85 and 1.75, respectively. This shows that the configuration of FIG. 5 is also more efficient compared to known configurations of FIG. 1 and FIG. 2

**Claims**

1. A method of synthesizing methanol, the method comprising:

   introducing a syngas reactor feed stream from a feed gas source comprising hydrogen gas ($H_2$) and carbon oxides of CO and $CO_2$ into a methanol synthesis reactor to form a reactor product stream comprising methanol, water, and unreacted carbon oxides of CO and $CO_2$ and hydrogen gas;
   separating methanol and water from the reactor product stream leaving an unreacted gas stream;
   providing at least one water gas shift (WGS) unit for converting CO and water to a WGS product stream of $CO_2$ and hydrogen gas and performing operation A and optionally operation B, wherein:

      A is introducing at least a portion of fresh syngas reactor feed from the feed gas source into the at least one WGS unit to provide a WGS product stream of $CO_2$ and hydrogen gas; and
      B is introducing at least a portion of the unreacted gas stream and steam into the at least one WGS unit for converting unreacted CO and water to a WGS product stream of $CO_2$ and hydrogen gas; and

   introducing at least a portion of the WGS product stream from operation A and optionally operation B into a hydrogen recovery unit for producing a hydrogen-rich stream, at least a portion of the hydrogen-rich stream being recycled and introduced into the methanol synthesis reactor as part of the reactor feed stream
   wherein said feed gas source comprises the product of catalytic partial oxidation of natural gas.

2. The method of claim 1, wherein: fresh reactor feed from the feed gas source has an M value of less than 2, wherein M is defined by Equation (1)

$$M = (F_{H2} - F_{CO2})/(F_{CO}+F_{CO2}) \qquad\qquad (1)$$

   where $F_{H2}$, $F_{CO2}$, and $F_{CO}$ are the molar flow rates of each of $H_2$, $CO_2$, and CO, respectively, in the syngas.

3. The method of claim 1, wherein: fresh reactor feed from the feed gas source has an M value of from 1.9 or less, wherein M is defined by Equation (1)

$$M = (F_{H2} - F_{CO2})/(F_{CO}+F_{CO2}) \qquad\qquad (1)$$

   where $F_{H2}$, $F_{CO2}$, and $F_{CO}$ are the molar flow rates of each of $H_2$, $CO_2$, and CO, respectively, in the syngas.

4. The method of claim 2, wherein: the reactor feed stream introduced into the reactor has an M value of from 4 or more.

5. The method of claim 1, wherein: all of the WGS product stream from the WGS unit is introduced into the hydrogen recovery unit inlet.

6. The method of claim 1, wherein: operation B is performed.

7. The method of claim 1, wherein: at least a portion of the unreacted gas stream is introduced directly into the hydrogen recovery unit without being introduced into a WGS unit.

8. The method of claim 1, wherein: at least a portion of the WGS product stream is introduced into the hydrogen recovery unit to form the hydrogen-rich stream and another portion of the WGS product stream is introduced into the methanol synthesis reactor without being introduced into the hydrogen recovery unit to form part of the reactor feed stream.

9. The method of claim 1, wherein:
   operations A and B are both performed using different WGS units.

10. The method of claim 1, wherein:
    operations A and B are both performed using the same WGS unit.

11. The method of claim 1, wherein:
    the feed gas source is the product of catalytic partial oxidation of natural gas.

12. The method of claim 1, wherein:
    the hydrogen recovery unit is a pressure swing adsorption unit.

13. The method of claim 1, wherein:
    the hydrogen recovery unit is a hydrogen separation membrane unit.

14. A system for the synthesis of methanol, the system comprising:

    a methanol synthesis reactor having a reactor inlet for receiving a syngas reactor feed stream from a feed gas source comprising hydrogen gas (H2) and carbon oxides of CO and CO2 that is converted in the reactor to a reactor product comprising methanol, water, and unreacted carbon oxides and hydrogen gas, the reactor having a reactor outlet for discharging a reactor product stream of the reactor product from the reactor;
    a separation unit having a separator inlet in fluid communication with the reactor outlet for receiving the reactor product stream and separating methanol and water from the reactor product stream to leave an unreacted gas stream that is discharged from the separation unit through an unreacted gas outlet;
    at least one water gas shift (WGS) unit for converting CO and water to a WGS product stream of CO2 and hydrogen gas, the at least one WGS unit having a WGS inlet and a WGS outlet: and
    a hydrogen recovery unit having a hydrogen recovery unit inlet that is in fluid communication with of the WGS outlet for receiving the WGS product stream from the WGS unit, the hydrogen recovery unit having a hydrogen recycle gas outlet that is in fluid communication with the reactor inlet for discharging a hydrogen-rich stream from the hydrogen recovery unit to the reactor inlet as a hydrogen gas recycle stream; and wherein the WGS inlet is positioned at location A and optionally location B, wherein;

        A is a position upstream of the methanol synthesis reactor wherein at least a portion of the fresh syngas reactor feed stream is delivered to the WGS inlet, the WGS outlet being in fluid communication with the hydrogen recovery unit inlet so that at least a portion of the WGS product stream is delivered to the hydrogen recovery unit; and
        B is a position downstream of the methanol synthesis reactor wherein the WGS inlet is in fluid communication with the unreacted gas outlet so that the WGS inlet receives at least a portion of the unreacted gas stream from the unreacted gas outlet, and wherein the WGS outlet is in fluid communication with the hydrogen recovery unit inlet so at least a portion of the WGS product stream from the WGS unit is delivered to the hydrogen recovery unit inlet.

15. The system of claim 14, wherein:
    the WGS inlet is positioned at B and the WGS outlet delivers all of the WGS product stream from the WGS unit to the hydrogen recovery unit inlet.

16. The system of claim 14, wherein:
    the WGS inlet is positioned at B and the WGS outlet is split into first and second outlets with the first outlet being in fluid communication with hydrogen gas recovery unit inlet and the second outlet being in fluid communication with the reactor inlet so that a portion of the WGS product stream is delivered to the hydrogen recovery unit inlet and another portion of the WGS product stream is delivered to the reactor inlet as part of the reactor feed stream.

17. The system of claim 14, wherein:
    the WGS inlet is positioned at both A and B.

18. The system of claim 17, wherein:

the at least one WGS unit is a single WGS unit having a WGS inlet positioned at both A and B.

19. The system of claim 14, wherein:
the WGS outlet of the at least one WGS unit is split into first and second outlets with the first outlet being in fluid communication with hydrogen gas recovery unit inlet and the second outlet being in fluid communication with the reactor inlet so that a portion of the WGS product stream is delivered to the hydrogen recovery unit inlet and another portion of the WGS product stream is delivered to the reactor inlet as part of the reactor feed stream.

20. The system of claim 14, wherein:
the hydrogen recovery unit has a hydrogen recovery unit inlet in fluid communication with the unreacted gas outlet of the separation unit for receiving the unreacted gas stream from the separation unit without being introduced into a WGS unit.

**Patentansprüche**

1. Verfahren zur Synthese von Methanol, wobei das Verfahren umfasst:

Einführen von einem Synthesegasreaktor-Einspeisungsstrom von einer Einspeisungsgasquelle, die Wasserstoffgas ($H_2$) und Kohlenstoffoxide von CO und $CO_2$ umfasst, in einen Methanolsynthesereaktor, um einen Reaktorproduktstrom zu bilden, der Methanol, Wasser und nicht umgesetzte Kohlenstoffoxide von CO und $CO_2$ und Wasserstoffgas umfasst;
Abtrennen von Methanol und Wasser aus dem Reaktorproduktstrom, wobei ein nicht umgesetzter Gasstrom zurückbleibt;
Bereitstellen von mindestens einer Wassergas-Shift (WGS) Einheit zum Umwandeln von CO und Wasser in einen WGS-Produktstrom aus $CO_2$ und Wasserstoffgas und Durchführen von Vorgang A und optional Vorgang B, wobei:

A mindestens einen Teil der frischen Synthesegasreaktoreinspeisung aus der Einspeisungsgasquelle in die mindestens eine WGS-Einheit einleitet, um einen WGS-Produktstrom aus $CO_2$ und Wasserstoffgas bereitzustellen; und
B mindestens einen Teil von dem nicht umgesetzten Gasstrom und Dampf in die mindestens eine WGS-Einheit zum Umwandeln von nicht umgesetztem CO und Wasser in einen WGS-Produktstrom aus $CO_2$ und Wasserstoffgas einleitet; und

Einleiten von mindestens einem Teil von dem WGS-Produktstrom aus Vorgang A und gegebenenfalls Vorgang B in eine Wasserstoff-Rückgewinnungs-Einheit zur Erzeugung von einem wasserstoffreichen Strom, wobei mindestens ein Teil von dem wasserstoffreichen Strom recycelt und in den Methanolsynthesereaktor als Teil von dem Reaktoreinspeisungsstrom eingeleitet wird
wobei die Einspeisungsgasquelle das Produkt der katalytischen Partialoxidation von Erdgas umfasst.

2. Verfahren nach Anspruch 1, wobei: frische Reaktoreinspeisung aus der Einspeisungsgasquelle einen M-Wert von weniger als 2 aufweist, wobei M durch Gleichung (1) definiert ist

$$M = (F_{H2} - F_{CO2})/(F_{CO} + F_{CO2}) \qquad (1)$$

wobei $F_{H2}$, $F_{CO2}$ und $F_{CO}$ die molaren Durchflussraten von jeweils $H_2$, $CO_2$ und CO im Synthesegas sind.

3. Verfahren nach Anspruch 1, wobei: frische Reaktoreinspeisung aus der Einspeisungsgasquelle einen M-Wert von 1,9 oder weniger aufweist, wobei M durch Gleichung (1) definiert ist

$$M = (F_{H2} - F_{CO2})/(F_{CO} + F_{CO2}) \qquad (1)$$

wobei $F_{H2}$, $F_{CO2}$ und $F_{CO}$ die molaren Durchflussraten von jeweils $H_2$, $CO_2$ und CO im Synthesegas sind.

4. Verfahren nach Anspruch 2, wobei: der in den Reaktor eingeleitete Reaktoreinspeisungsstrom einen M-Wert von 4 oder mehr aufweist.

5. Verfahren nach Anspruch 1, wobei: der gesamte WGS-Produktstrom aus der WGS-Einheit in den Einlass der Wasserstoffrückgewinnungseinheit eingeleitet wird.

6. Verfahren nach Anspruch 1, wobei: Vorgang B durchgeführt wird.

7. Verfahren nach Anspruch 1, wobei: mindestens ein Teil von dem nicht umgesetzten Gasstrom direkt in die Wasserstoffrückgewinnungseinheit eingeleitet wird, ohne in eine WGS-Einheit eingeleitet zu werden.

8. Verfahren nach Anspruch 1, wobei: mindestens ein Teil von dem WGS-Produktstrom in die Wasserstoffrückgewinnungseinheit eingeleitet wird, um den wasserstoffreichen Strom zu bilden, und ein anderer Teil von dem WGS-Produktstrom in den Methanolsynthesereaktor eingeleitet wird, ohne in die Wasserstoffrückgewinnungseinheit eingeleitet zu werden, um einen Teil von dem Reaktoreinspeisungsstrom zu bilden.

9. Verfahren nach Anspruch 1, wobei:
die Vorgänge A und B beide mit unterschiedlichen WGS-Einheiten durchgeführt werden.

10. Verfahren nach Anspruch 1, wobei:
die Vorgänge A und B beide mit der gleichen WGS-Einheit durchgeführt werden.

11. Verfahren nach Anspruch 1, wobei:
die Einspeisungsgasquelle das Produkt der katalytischen Partialoxidation von Erdgas ist.

12. Verfahren nach Anspruch 1, wobei:
die Wasserstoffrückgewinnungseinheit eine Druckwechseladsorptionseinheit ist.

13. Verfahren nach Anspruch 1, wobei:
die Wasserstoffrückgewinnungseinheit eine Wasserstoffseparationsmembraneinheit ist.

14. System zur Synthese von Methanol, wobei das System umfasst:

einen Methanolsynthesereaktor mit einem Reaktoreinlass zur Aufnahme von einem Synthesegasreaktor-Einspeisungsstrom von einer Einspeisungsgasquelle, die Wasserstoffgas (H2) und Kohlenstoffoxide von CO und CO2 umfasst, das in dem Reaktor in ein Reaktorprodukt umgewandelt wird, das Methanol, Wasser und nicht umgesetzte Kohlenstoffoxide und Wasserstoffgas umfasst, wobei der Reaktor einen Reaktorauslass zum Ablassen von einem Reaktorproduktstrom von dem Reaktorprodukt aus dem Reaktor aufweist;
eine Separationseinheit mit einem Separatoreinlass, der in Fluidverbindung mit dem Reaktorauslass steht, um den Reaktorproduktstrom aufzunehmen und Methanol und Wasser aus dem Reaktorproduktstrom abzutrennen, so dass ein nicht umgesetzter Gasstrom zurückbleibt, der aus der Separationseinheit durch einen Auslass für nicht umgesetztes Gas abgeleitet wird;
mindestens eine Wassergas-Shift (WGS) Einheit zum Umwandeln von CO und Wasser in einen WGS-Produktstrom aus CO2 und Wasserstoffgas, wobei die mindestens eine WGS-Einheit einen WGS-Einlass und einen WGS-Auslass aufweist; und
eine Wasserstoff-Rückgewinnungseinheit mit einem Einlass der Wasserstoff-Rückgewinnungseinheit, der in Fluidverbindung mit dem WGS-Auslass steht, um den WGS-Produktstrom von der WGS-Einheit zu empfangen, wobei die Wasserstoff-Rückgewinnungseinheit einen Auslass für Wasserstoff-Recyclegas aufweist, der in Fluidverbindung mit dem Reaktoreinlass steht, um einen wasserstoffreichen Strom von der Wasserstoff-Rückgewinnungseinheit zum Reaktoreinlass als Wasserstoffgas-Recyclestrom abzugeben; und wobei
der WGS-Einlass an Ort A und optional an Ort B positioniert ist, wobei;

A eine Position stromaufwärts von dem Methanolsynthesereaktor ist, in der mindestens ein Teil von dem frischen Synthesegasreaktor-Einspeisungsstrom dem WGS-Einlass zugeführt wird, wobei der WGS-Auslass in Fluidverbindung mit dem Einlass der Wasserstoffrückgewinnungseinheit ist, so dass mindestens ein Teil von dem WGS-Produktstrom der Wasserstoffrückgewinnungseinheit zugeführt wird; und
B eine Position stromabwärts von dem Methanolsynthesereaktor ist, wobei der WGS-Einlass in Fluidverbindung mit dem Auslass für nicht umgesetztes Gas steht, so dass der WGS-Einlass mindestens einen Teil von dem nicht umgesetzten Gasstrom vom Auslass für nicht umgesetztes Gas erhält, und wobei der WGS-Auslass in Fluidverbindung mit dem Einlass der Wasserstoffrückgewinnungseinheit steht, so dass mindestens ein Teil von dem WGS-Produktstrom von der WGS-Einheit an den Einlass der Wasserstoffrückgewinnungseinheit geliefert wird.

15. System nach Anspruch 14, wobei:
der WGS-Einlass sich bei B befindet und der WGS-Auslass den gesamten WGS-Produktstrom aus der WGS-Einheit zum Einlass der Wasserstoffrückgewinnungseinheit führt.

16. System nach Anspruch 14, wobei:
der WGS-Einlass bei B angeordnet ist und der WGS-Auslass in einen ersten und einen zweiten Auslass aufgeteilt ist, wobei der erste Auslass in Fluidverbindung mit dem Einlass der Wasserstoffgas-Rückgewinnungseinheit und der zweite Auslass in Fluidverbindung mit dem Reaktoreinlass steht, so dass ein Teil von dem WGS-Produktstrom dem Einlass der Wasserstoff-Rückgewinnungseinheit und ein anderer Teil von dem WGS-Produktstrom dem Reaktoreinlass als Teil von dem Reaktor-Einspeisungsstrom zugeführt wird.

17. System nach Anspruch 14, wobei:
der WGS-Eingang sich sowohl bei A als auch bei B befindet.

18. System nach Anspruch 17, wobei:
die mindestens eine WGS-Einheit eine einzelne WGS-Einheit ist mit einem WGS-Einlass, der sowohl an A und B angeordnet ist.

19. System nach Anspruch 14, wobei:
der WGS-Auslass der mindestens einen WGS-Einheit in einen ersten und einen zweiten Auslass aufgeteilt ist, wobei der erste Auslass in Fluidverbindung mit dem Einlass der Wasserstoffgasrückgewinnungseinheit und der zweite Auslass in Fluidverbindung mit dem Reaktoreinlass steht, so dass ein Teil von dem WGS-Produktstrom dem Einlass der Wasserstoffrückgewinnungseinheit zugeführt wird und ein anderer Teil von dem WGS-Produktstrom dem Reaktoreinlass als Teil von dem Reaktoreinspeisungsstrom zugeführt wird.

20. System nach Anspruch 14, wobei:
die Wasserstoffrückgewinnungseinheit einen Einlass für die Wasserstoffrückgewinnungseinheit aufweist, der in Fluidverbindung mit dem Auslass für nicht umgesetztes Gas der Separationseinheit steht, um den Strom von dem nicht ungesetztem Gas aus der Separationseinheit zu empfangen, ohne in eine WGS-Einheit eingeleitet zu werden.

**Revendications**

1. Procédé de synthèse de méthanol, le procédé comprenant les étapes consistant à:

introduire un courant de charge de réacteur de gaz de synthèse provenant d'une source de gaz d'alimentation comprenant du gaz hydrogène ($H_2$) et des oxydes de carbone constitués de CO et $CO_2$ dans un réacteur de synthèse de méthanol pour former un courant de produit de réacteur comprenant du méthanol, de l'eau et du gaz hydrogène et des oxydes carbone CO et $CO_2$ n'ayant pas réagi;
séparer le méthanol et l'eau du courant de produit de réacteur, en laissant un courant de gaz n'ayant pas réagi;
disposer au moins une unité de conversion de gaz à l'eau (WGS) pour convertir le CO et l'eau en un courant de produit de WGS composé de $CO_2$ et gaz hydrogène et effectuer une opération A et en option une opération B,

A consistant à introduire au moins une partie de charge neuve de réacteur de gaz de synthèse provenant de la source de gaz d'alimentation dans ladite au moins une unité de WGS pour produire un courant de produit de WGS composé de $CO_2$ et gaz hydrogène; et
B consistant à introduire au moins une partie du courant de gaz n'ayant pas réagi et de la vapeur dans ladite au moins une unité de WGS pour convertir l'eau et le CO n'ayant pas réagi en un courant de produit de WGS composé de $CO_2$ et gaz hydrogène; et

introduire au moins une partie du courant de produit de WGS provenant de l'opération A et en option de l'opération B dans une unité de récupération d'hydrogène pour produire un courant riche en hydrogène, au moins une partie du courant riche en hydrogène étant recyclée et introduite dans le réacteur de synthèse de méthanol en tant que partie du courant de charge du réacteur

dans lequel ladite source de gaz d'alimentation comprend le produit d'oxydation catalytique partielle de gaz naturel.

2. Procédé selon la revendication 1, dans lequel: la charge neuve du réacteur, provenant de la source de gaz d'alimentation, a une valeur M inférieure à 2, M étant définie par l'équation (1)

$$M = (F_{H2} - F_{CO2})/(F_{CO} + F_{CO2}) \qquad (1)$$

où $F_{H2}$, $F_{CO2}$, et $F_{CO}$ sont les débits molaires de chacun de $H_2$, CO, et CO, respectivement, dans le gaz de synthèse.

3. Procédé selon la revendication 1, dans lequel: la charge neuve du réacteur, provenant de la source de gaz d'alimentation, a une valeur M de 1,9 ou moins, M étant définie par l'équation (1)

$$M = (F_{H2} - F_{CO2})/(F_{CO} + F_{CO2}) \qquad (1)$$

où $F_{H2}$, $F_{CO2}$, et $F_{CO}$ sont les débits molaires de chacun de $H_2$, CO, et CO, respectivement, dans le gaz de synthèse.

4. Procédé selon la revendication 2, dans lequel: le courant de charge du réacteur, introduit dans le réacteur, a une valeur de 4 ou plus.

5. Procédé selon la revendication 1, dans lequel: la totalité du courant de produit de WGS provenant de l'unité de WGS est introduite dans l'entrée de l'unité de récupération d'hydrogène.

6. Procédé selon la revendication 1, dans lequel: l'opération B est effectuée.

7. Procédé selon la revendication 1, dans lequel: au moins une partie du courant de gaz n'ayant pas réagi est introduite directement dans l'unité de récupération d'hydrogène, sans être introduite dans une unité de WGS.

8. Procédé selon la revendication 1, dans lequel: au moins une partie du courant de produit de WGS est introduite dans l'unité de récupération d'hydrogène pour former le courant riche en hydrogène et une autre partie du courant de produit de WGS est introduite dans le réacteur de synthèse de méthanol sans être introduite dans l'unité de récupération d'hydrogène pour former une partie du courant de charge du réacteur.

9. Procédé selon la revendication 1, dans lequel:
les opérations A et B sont l'une et l'autre effectuées en utilisant des unités de WGS différentes.

10. Procédé selon la revendication 1, dans lequel:
les opérations A et B sont l'une et l'autre effectuées en utilisant la même unité de WGS.

11. Procédé selon la revendication 1, dans lequel:
la source de gaz d'alimentation est le produit d'oxydation catalytique partielle de gaz naturel.

12. Procédé selon la revendication 1, dans lequel:
l'unité de récupération d'hydrogène est une unité d'adsorption à pression modulée.

13. Procédé selon la revendication 1, dans lequel:
l'unité de récupération d'hydrogène est une unité de membrane de séparation d'hydrogène.

14. Système pour la synthèse de méthanol, le système comprenant:

un réacteur de synthèse de méthanol comportant une entrée de réacteur destinée à recevoir un courant de charge de réacteur de gaz de synthèse provenant d'une source de gaz d'alimentation comprenant du gaz hydrogène ($H_2$) et des oxydes de carbone constitués de CO et $CO_2$ qui est converti dans le réacteur en un produit de réacteur comprenant du méthanol, de l'eau, et du gaz hydrogène et des oxydes de carbone n'ayant pas réagi, le réacteur comportant une sortie de réacteur destinée à décharger un courant de produit de réacteur du produit de réacteur, provenant du réacteur;

une unité de séparation comportant une entrée de séparateur en communication fluidique avec la sortie de réacteur, destinée à recevoir le courant de produit de réacteur, et séparant le méthanol et l'eau du courant de produit de réacteur pour laisser un courant de gaz n'ayant pas réagi qui est déchargé de l'unité de séparation à travers une sortie de gaz n'ayant pas réagi;

au moins un unité de conversion de gaz à l'eau (WGS) destinée à convertir le CO et l'eau en un courant de produit de WGS composé de $CO_2$ et gaz hydrogène, ladite au moins une unité de WGS comportant une entrée de WGS et une sortie de WGS; et

une unité de récupération d'hydrogène comportant une entrée d'unité de récupération d'hydrogène qui est en communication fluidique avec la sortie de WGS pour recevoir le courant de produit de WGS provenant de l'unité de WGS, l'unité de récupération d'hydrogène comportant une sortie de gaz de recyclage d'hydrogène qui est en communication fluidique avec l'entrée de réacteur pour décharger un courant riche en hydrogène, provenant de l'unité de récupération d'hydrogène, dans l'entrée de réacteur, en tant qu'un courant de recyclage de gaz hydrogène; et dans lequel

l'entrée de WGS est disposée à l'emplacement A et en option à l'emplacement B;

A étant une position en amont du réacteur de synthèse de méthanol où au moins une partie du courant de charge neuve du réacteur de gaz de synthèse est introduite dans l'entrée de WGS, la sortie de WGS étant en communication fluidique avec l'entrée de l'unité de récupération d'hydrogène, de sorte qu'au moins une partie du courant de produit de WGS est amenée à l'unité de récupération d'hydrogène; et

B étant une position en aval du réacteur de synthèse de méthanol où l'entrée de WGS est en communication fluidique avec la sortie de gaz n'ayant pas réagi, de sorte que l'entrée de WGS reçoit au moins une partie du courant de gaz n'ayant pas réagi, provenant de la sortie de gaz n'ayant pas réagi, et où la sortie de WGS est en communication fluidique avec l'entrée de l'unité de récupération d'hydrogène, de sorte qu'au moins une partie du courant de produit de WGS, provenant de l'unité de WGS, est amenée à l'entrée de l'unité de récupération d'hydrogène.

15. Système selon la revendication 14, dans lequel:
l'entrée de WGS est disposée en B et la sortie de WGS envoie à l'entrée de l'unité de récupération d'hydrogène la totalité du courant de produit de WGS provenant de l'unité de WGS.

16. Système selon la revendication 14, dans lequel
l'entrée de WGS est disposée en B et la sortie de WGS est divisée en première et seconde sorties, la première sortie étant en communication fluidique avec l'entrée de l'unité de récupération d'hydrogène et la seconde sortie étant en communication fluidique avec l'entrée du réacteur, de sorte qu'une partie du courant de produit de WGS est envoyée à l'entrée de l'unité de récupération d'hydrogène et une autre partie du courant de produit de WGS est envoyée à l'entrée du réacteur en tant que partie du courant de charge du réacteur.

17. Système selon la revendication 14, dans lequel:
l'entrée de WGS est disposée à la fois en A et B.

18. Système selon la revendication 17, dans lequel:
ladite au moins une unité de WGS est une unité de WGS unique comportant une entrée de WGS disposée à la fois en A et B.

19. Système selon la revendication 14, dans lequel:
la sortie de WGS de ladite au moins une unité de WGS est divisée en première et seconde sorties, la première sortie étant en communication fluidique avec l'entrée de l'unité de récupération d'hydrogène et la seconde sortie étant en communication fluidique avec l'entrée du réacteur, de sorte qu'une partie du courant de produit de WGS est envoyée à l'entrée de l'unité de récupération d'hydrogène et une autre partie du courant de produit de WGS est envoyée à l'entrée du réacteur, en tant que partie du courant de charge du réacteur.

20. Système selon la revendication 14, dans lequel:
l'unité de récupération d'hydrogène comporte une entrée d'unité de récupération d'hydrogène en communication fluidique avec la sortie de gaz n'ayant pas réagi de l'unité de séparation pour recevoir le courant de gaz n'ayant pas réagi provenant de l'unité de séparation sans être introduit dans une unité de WGS.

FIG. 1
(PRIOR ART)

FIG. 2
(PRIOR ART)

EP 3 844 135 B1

FIG. 3

EP 3 844 135 B1

FIG. 4

UNREACTED, HYDROGEN LEAN PURGE GAS

62

STEAM

70

60

HYDROGEN RECOVERY

74

72

52

WATER GAS SHIFT

66

76

64

56

58

METHANOL, WATER

30

84

32

UNREACTED GAS

36

34

82

28

26

20

RECYCLE GAS

22

18

METHANOL SYNTHESIS REACTOR

68

16

FIG. 5

HYDROGEN RICH RECYCLE GAS

90

14

REACTOR FEED

12

FRESH SYNGAS

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9969666 B